# EUROPEAN PATENT SPECIFICATION

(11) **EP 1 979 024 B1**
(45) Date of publication and mention of the grant of the patent: **02.11.2016**
(21) Application number: 07762954.1
(22) Date of filing: 24.01.2007
(51) Int. Cl.: A61B 17/34, A61M 5/158, A61M 5/165, A61M 39/10, A61M 5/32

(54) **SAFETY NEEDLE ASSEMBLY WITH CORRECT MEDICATION CONNECTION**
SICHERHEITSNADELANORDNUNG MIT RICHTIGER MEDIKAMENTENVERBINDUNG
ENSEMBLE AIGUILLE DE SECURITE AVEC RACCORD CORRECT POUR MEDICATION

(30) Priority: 31.01.2006 US 342620
(43) Date of publication of application: 15.10.2008
(73) Proprietor: Smiths Medical ASD, Inc., Plymouth, MN 55442 (US)
(72) Inventor: ZIMAN, Lynn, A., Swanzey, NH 03446 (US); NGUYEN, Steven, Huu, North Brunswick, NJ 08902 (US)
(74) Representative: Tranter, Andrew David
(86) International application number: PCT/US2007/002004
(87) International publication number: WO 2007/089531

(56) References cited:
- US-A1- 2003 105 428
- US-A1- 2004 201 216
- US-A1- 2004 201 216
- US-B1- 6 402 207
- US-B1- 6 428 514
- US-B1- 6 612 624

## Description

### Field of the Invention

The present invention relates to a needle assembly, such as for example a spinal or epidural needle assembly, and more particularly to a needle assembly that is designed to mate with an adapter for correct connection to a particular medication store or line.

### Background of the Invention

To prevent mis-connection of a fluid line or a fluid store to a needle, the prior art teaches the use of a two-part connector with complementary configured opposing surfaces. Such two-part connector ensures that one line from one portion of the connector would not be wrongly connected to a different medication line, and is disclosed in U.S. patent 6,612,624 and its parent U.S. patent 6,402,207.

An adapter that may be used in place of the connector disclosed in the '624 patent is disclosed in U.S. application No. 10/915,574 filed on August 11, 2004, assigned to same assignee as the instant application.

As the connector of the '624 patent requires that there be a two-part connection, in order to use a needle, there is the requirement that a fluid line that converts the input of the needle be added. This is an inconvenience, not to mention the consumption of valuable time, in a medical environment where potentially every second counts.

### Summary of the Present Invention

The instant invention needle assembly includes a needle that has a needle hub specifically configured to have formation(s) thereon that allows it to be connected only to one end of an adapter, which is configured to have a complementary configuration that allows it and the needle hub to readily mate with each other. The other end of the adapter has a conventional receptacle end, which may be in the form of a luer that allows it to be connected to a conventional luer fitted medication store, such as for example a syringe or a medication fluid line. The hub of the needle assembly of the instant invention, as it is configured to have a particular configuration, is not fittable to a conventional luer. Accordingly, the needle assembly could not be mistakenly connected to a fluid store that may contain medicament that, if injected to a patient, may cause harm to the patient.

As the needle may need to be removed from the fluid store, and/or additional medication be provided to the patient, to ensure that the proper medicament is provided to the patient, the adapter that connects the needle to the appropriate medicament store (or fluid tine) has a lock mechanism that prevents the removal of the fluid store once the correct fluid store is connected to the adapter. This ensures that no more medication than necessary be injected to the patient, and also that the correct medicament be provided to the patient from the correct mating of the medicament store and the needle.

The lock mechanism for the instant invention is an integral part of the adapter in that the adapter is made up of two components, namely an adapter core and a shroud that fits about the adapter core. The adapter core is fitted to the shroud during manufacturing. The adapter core has a pair of pawls formed at a substantially central portion of its circumferential outer surface or wall. These pawls act against ramped stops formed on the interior circumferential surface or wall of the shroud, when the adapter core and the shroud are rotated relative to each other. Once coupled to the adapter, the fluid store (or fluid line) may no longer be removed from the adapter, as rotation in the direction that ordinarily would have uncoupled the receptacle end of the fluid store from the adapter would cause the receptacle end of the adapter to rotate in unison with the fluid store, thereby preventing the receptacle end of the fluid store and the receptacle end of the adapter from disengaging. As a result, whatever medicament stored in the fluid store that is meant to be used with the needle, which would only mate with the particular adapter, could only be used with the needle, thereby preventing any possible mis-connection of a different medicament container to the needle, which may still be inserted to the patient.

The instant invention therefore relates to an apparatus that comprises a needle assembly that includes a hub having a given configuration at its receptacle end and a needle extending from its closed end, and an adapter having a first end with a first configuration complementary to the given configuration for mating with the hub at its receptacle end. The adapter further has a second end with a second configuration so that a fluid store, or a fluid line, that has a receptacle end with a configuration complementary to the second configuration is adaptable to mate with the second end of the adapter.

The instant invention also relates to a combination in which a needle assembly having a needle hub with a receptacle end of a given configuration and a needle extending from its closed end is combined with an adapter having a first end with a configuration complementary to the given configuration so that the needle hub and the first end of the adapter are readily matable with each other. The adapter has a second end with a second configuration that allows the adapter to be coupled to the receptacle end of a fluid store, or a fluid line. The adapter further acts as a locking mechanism to prevent the fluid store (or the fluid line), once coupled to the adapter, from rotating in a direction relative to the adapter that allows the fluid store from being uncoupled from the adapter.

The instant invention further relates to a method of coupling a needle assembly including a hub having a receptacle end and a needle extending from its closed end to an appropriate medicament fluid store or fluid line. The method includes the steps of: a) effecting a given configuration at the receptacle end of the hub; b) providing an adapter with a first end having a first configuration complementary to the given configuration for mating with the hub at its receptacle end; c) effecting a second configuration to a second send of the adapter; and d) providing a fluid store or a fluid line having a receptacle end with a configuration complementary to the second configuration for mating with the second end of the adapter.

### Brief Description of the Figures

The present invention will become apparent and the invention itself will be best understood with reference to the following description of the present invention taken in conjunction with the accompanying drawings, wherein:
Fig. 1 illustrates a needle of the instant invention with an adapter and a fluid store;
Fig. 2 is a view illustrating the disassembled components of the adapter, as it relates to the needle;
Figs. 3a and 3b are side views of the needle assembly of the instant invention;
Figs. 4a-4f are different views of the adapter core component of the adapter;
Figs. 5a-5g are different views of the shroud component of the adapter; and
Fig. 6 shows a filter interposed between the adapter and the needle assembly.

### Detailed Description of the Invention

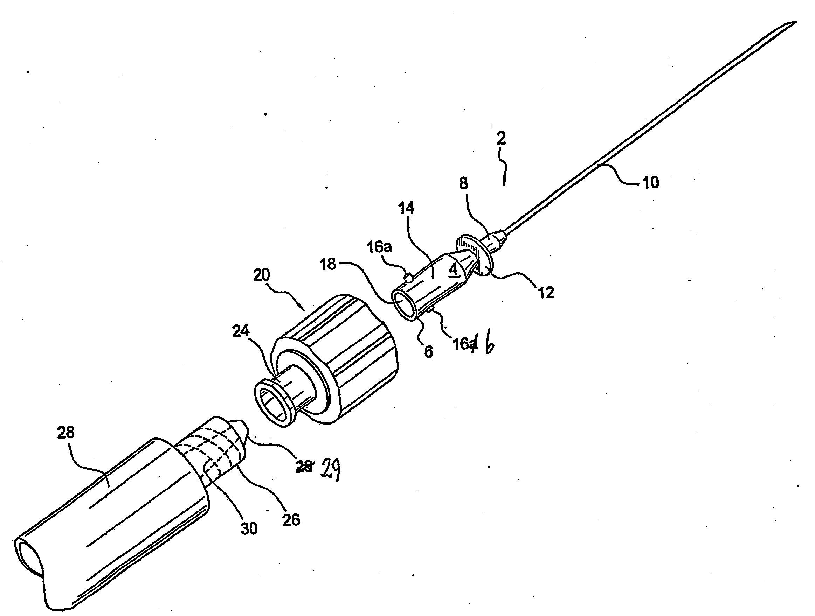

The instant invention, as shown in Fig. 1, includes a needle assembly 2 that has a needle hub 4. Needle hub 4 has a receptacle end 6 and a closed end 8 from which a needle 10 extends. Needle 10 may be a conventional needle, but for the instant embodiment is an epidural or a spinal needle for insertion to a patient. As shown, the closed end 8 is separated from the rest of needle hub 4 by a flange 12.

Receptacle end of needle hub 4 comprises an elongate cylindrical portion whereat a particular formation, or formations, are effected during the manufacturing process for providing a particular or given configuration that is unique to the needle assembly. For the embodiment shown, there are two protrusions 16a and 16b formed at opposite sides of the receptacle end 6. These protrusions, along with the cross sectional dimension of receptacle end 6, provide the given configuration for needle hub 4 of the needle assembly 2. The thickness of the circumferential wall of the elongate cylindrical portion 14 is dimensioned to provide a further attribute of the configuration of needle hub 4, i.e., configuring the cross section of opening 18 at receptacle end 6 and the through passage into needle hub 4. Opening 18 is of a sufficient dimension to accept the receptacle end of an adapter 20 to which needle hub 4 of the needle assembly 2 mates with. Protrusions 16a and 16b are formed on the receptacle end 6 of needle hub 4 a particular distance from opening 18 as part of the formation for effecting the proper mating with adapter 20, more specifically with the receptacle end thereof and the shroud component of adapter 20, to be discussed later.

As further shown in Fig. 1, adapter 20 has another receptacle end 24, which may be configured as a conventional luer end for mating with the conventional luer en d 26 of a fluid store, such as for example a syringe 28. In place of a fluid store, a fluid line having fitted to its end a luer such as 26 is also contemplated. Although the receptacle end 26 of syringe 28 is able to mate with receptacle end 24 of a adapter 20, it could not mate with the receptacle end 6 of needle assembly 2. Receptacle end 26 of syringe 28 has a male receptacle end 29 and is internally threaded, as represented by the dotted threaded line 30.

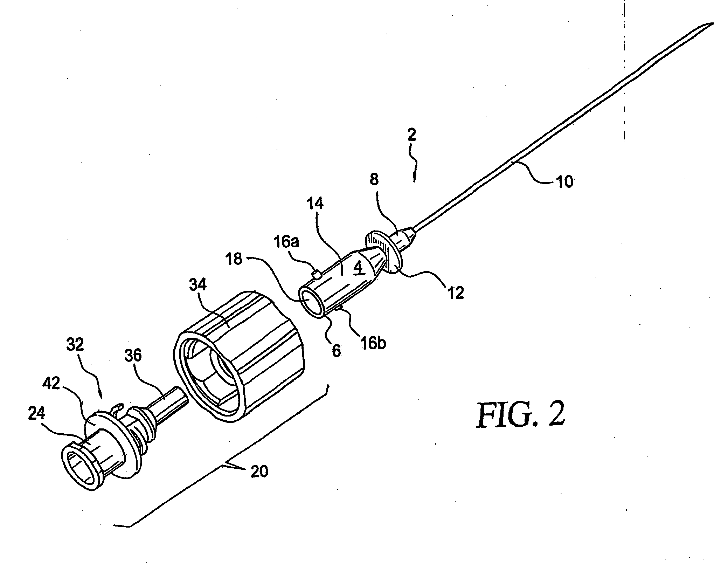

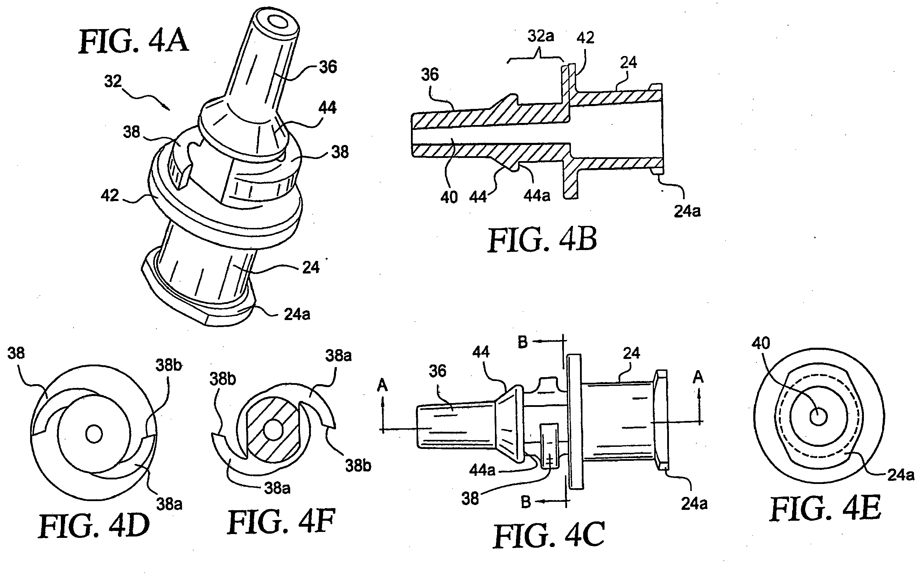

Fig. 2 shows the different components of adapter 20 in relation to needle assembly 2. As shown, adapter 20 comprises an adapter core 32 and a shroud 34 that is adapted to fit about core 32. As mentioned previously, adapter core 32 has a receptacle end 24, which may also be referred to as its second or distal end. In addition, core 32 has a first receptacle end 36, or a first or proximal end, for mating with receptacle end 6 of needle hub 4. The first end 36 and the second end 24 of adapter core 32 are connected, as adapter core 32 is a single molded piece, by a through passage 40 (Fig. 4b) that extends from the opening at first end 36 to the opening at second end 24. Shroud 34 and adapter core 32 are separate pieces that may be molded from conventional medical plastics.

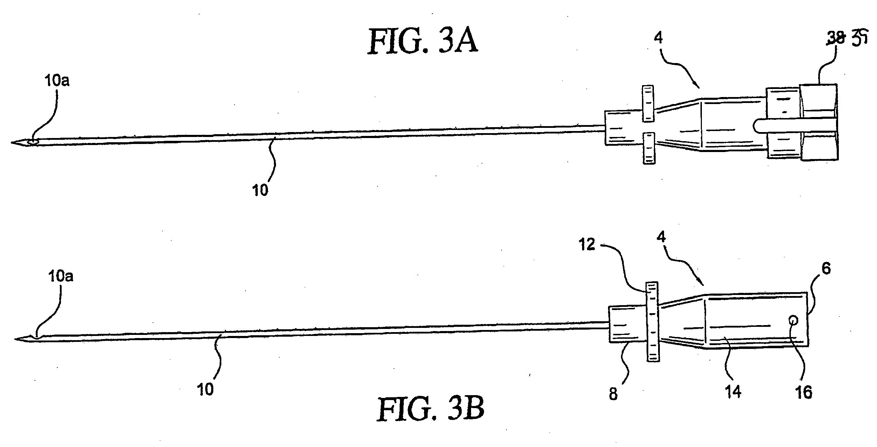

Needle assembly is shown in side views 3a and 3b. Fig. 3a shows the receptacle end 6 of needle hub 4 of the needle assembly 2 being covered by a cap 39 that has extending therefrom a thin cannula (not shown) inserted within needle 10 to prevent coring, or the blocking of the needle opening 10a, when needle 10 is inserted to the patient. It is only after needle 10 has been properly inserted into the patient would the user remove cap 38, and therefore the cannula inserted in needle 10. At which time, fluid may pass between needle 10 and the patient through opening 10a at the tip of needle 10.

Adapter core 32 of adapter 20 of the instant invention, as best shown in Figs. 4a-4f, has an elongate conical first end 36 and a cylindrical second end 24, which has at its end flanges 24a for forming a conventional luer connection. As best shown in Figs. 4a, 4d and 4f, at the proximate mid-section of adapter core 32 there are two integral pawls 38 formed at the outer circumferential surface or wall of core 32. These pawls each have a semi-ramped portion 38a and an end stop 38b, as best shown in Fig. 4f. As best shown in Fig. 4b, a through passage 40 extends from the opening at first end 36 to the opening at second end 24. A flange 42 that separates the first end from the second end provides a back stop, when adapter core 36 is fitted into shroud 34 (or conversely shroud 34 being fitted about adapter core 32), as shown in Fig. 1. A tapered circumferential portion 44 enables adapter core 32 to be inserted to shroud 34, and be fixedly but rotatably retained within shroud 34.

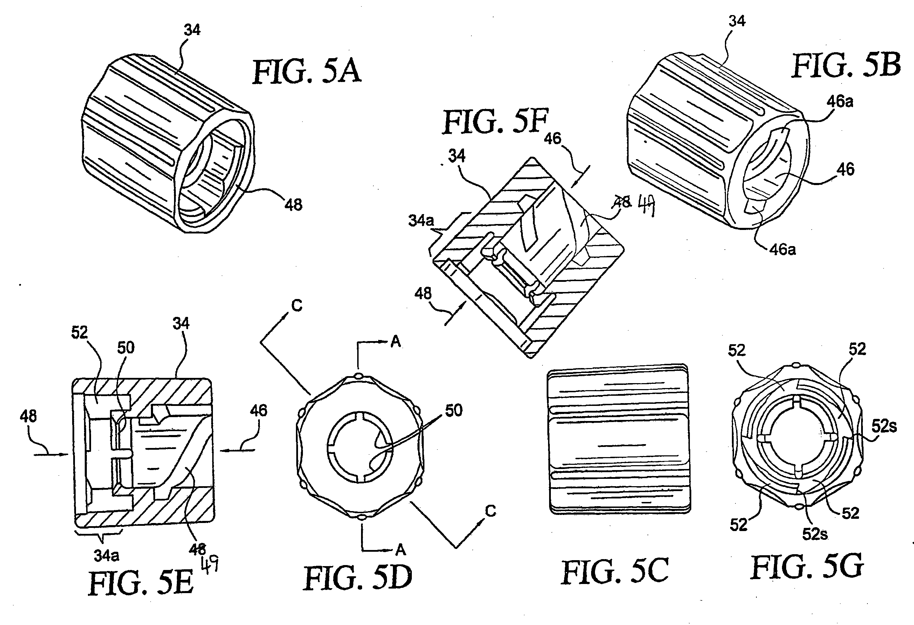

Shroud 34 is shown in Figs. 5a-5g to have a first opening 46 and a second opening 48. Opening 46 is fabricated to have a configuration that is complementary to the configuration of the formation at receptacle end 6 of needle assembly 2, as discussed above. In particular, as shown in Fig. 5b, opening 46 of shroud 34 has a formation in the shape of a circle, but with two side inlets or channels 46a that allow the corresponding protrusions 16 of needle hub 4 to pass through. As the shroud is formed with an internal thread 49, receptacle end 6 of needle hub 4, once inserted to opening 46, may be secured to shroud 34 by the relative rotation of hub 4 and shroud 34, so that protrusions 16 of needle hub may travel along the internal thread of shroud 34, and receptacle end 6 via its opening 18 be more fully mated-with the receptacle end 36 of adapter core 32. Once fully threaded, needle hub 4 is securely mated to adapter 20.

Adapter core 32 is pressedly fitted to shroud 34 in the direction as shown by directional arrow 48 in Fig. 5e and 5f. The portion of adapter core 32, designated 32a in Fig. 4b, is fitted and held by shroud 34 at portion 34a, as shown in Figs. 5e and 5f. A. number of extensions 50 circumferentially spaced at the interior wall of shroud 34, due to their inherent plastics elasticity, would allow portion 44 of adapter core 32 to pass through, as the latter's front is tapered, but would prevent the same from coming back out of shroud 34, as the circular flat backside 44a of portion 44 is biasedly held by extensions 50. As a result, once adapter core 32 is fitted into shroud 34, it cannot be removed therefrom.

Also provided in shroud 34 are a number of ramped stops 52, as best shown in Fig. 5g, that prevent adapter core 32 from rotating in a direction where the stop surfaces 52s would bias against pawl stops 38b. In practice, if a user were to hold adapter 20 by its receptacle end 24 while attempting to rotate shroud 34, she could only rotate shroud 34 in a counterclockwise direction. Any attempt to rotate shroud 34 relative to adapter core 32 in the clockwise direction would also cause adapter 32 to rotate in unison with shroud 34.

In operation, by rotating luer end 24 of adapter 20 relative to the luer end of a fluid store, such as for example syringe 28 shown in Fig. 1, in a clockwise direction, adapter 20 can readily be coupled to the fluid store. However, any attempt to remove adapter 20 from syringe 28, by for example rotating adapter 2 (more specifically shroud 34) counterclockwise relative to syringe 28 would fail, as adapter core 32 would move (rotation or non-rotation) in unison with syringe 28, while the movement of shroud 34 (non-rotation or rotation) would be independent of the unified movement of adapter core 32 and syringe 28. As a result, once syririge 28 is coupled to adapter 20, it remains coupled thereto, thereby eliminating the real possibility that a different syringe may be mis-connected to needle assembly 2, which hub has a configuration that could only mate with the complementary configuration at receptacle end 36 of adapter 20.

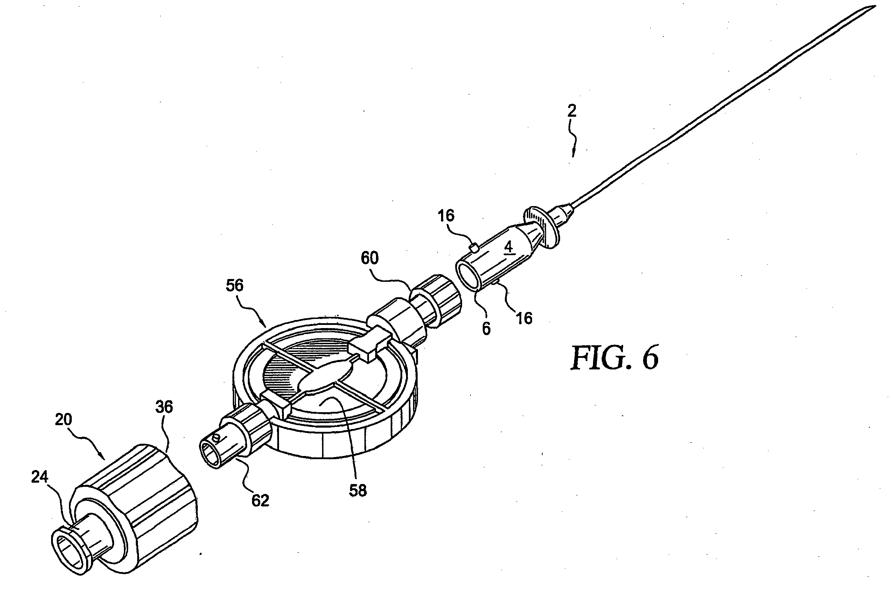

Another aspect of the instant invention is shown in Fig. 6. There a filter device 56 is shown to be interposedly connectable to adapter 20 and needle assembly 2. Filter device 56, in addition to having a filter element 58, has a first end 60 that has a configuration that is complementary to the configuration of the receptacle end 6 of needle hub 4 of the needle assembly 2. In other words, the configuration of the receptacle end 60 for filter device 56 is the same as the configuration of the receptacle end 36 of adapter 20. Further, filter device 56 has a second end 62 for mating with adapter 20 that has a configuration that is the same as the configuration of receptacle end 6 of needle hub 4. Once fully connected to both needle assembly 2 and adapter 20, filter device 56 filters the fluid passing between the needle assembly 2 and adapter 20, and of course the fluid store that is connected to end 24 of adapter 20. The filter element 58 of filter device 56 may be any conventional medical filter that is adaptable to filter out undesirable particles or elements that may be in the fluid.

## Claims

1. Apparatus comprising:
a needle assembly (2) including a hub (4) having a particular formation effected during the manufacturing process for providing a given configuration at its receptacle end (6) and a needle (10) extending from its closed end (8);
an adapter (20) having a first end (36 and 46, 46a) with a first configuration complementary to said given configuration for mating with said hub at its receptacle end, said adapter having a second end (24) with a second configuration, the first and second ends are connected by a through passage (40) through a single molded piece (32) of said adapter; and
a fluid store (28) or a fluid line having a receptacle end (29) with a configuration complementary to said second configuration for mating with the second end of said adapter;
wherein said adapter comprises a locking mechanism (38, 52) to prevent the removal of the receptacle end (29) of said fluid store (28) or fluid line from the second end (24) of said adapter once said fluid store or fluid line and said adapter have been mated to each other as rotation in the direction that ordinarily would have uncoupled the receptacle end of said fluid store from the second end of said adapter would cause the second end of said adapter to rotate in unison with the receptacle end of said fluid store or fluid line to thereby prevent said fluid store or fluid line and said adapter from disengaging.

2. Apparatus of claim 1, wherein the receptacle end of said hub has an integral formation (16a, 16b) for forming said given configuration so that said hub is adapted to mate only with a counterpart receptacle end that has a configuration complementary to said given configuration.

3. Apparatus of claim 2, wherein said integral formation of the receptacle end of said hub comprise two protrusions extending from the receptacle end of said hub.

4. Apparatus of claim 1, wherein said adapter comprises a shroud (34) fitted about an adapter core (32), the adapter core being the single molded piece, the first and second ends (24, 36) of said adapter formed at opposite ends of said adapter core, wherein the locking mechanism including at least one pawl (38) formed on a circumferential outer surface of said adapter core for coacting against ramped stops (42) formed circumferentially along an interior surface of said shroud so that once said fluid store or fluid line is mated to the second end of said adapter, said fluid store or fluid line and said adapter cannot be uncoupled from each other.

5. Apparatus of claim 1, wherein said needle assembly comprises an epidural needle.

6. Apparatus of claim 1, wherein said adapter comprises an adapter core having a through channel (40) and a shroud fitted about said adapter core, the first end (36) of said adapter core being a tapered inner receptacle end that forms the first end of said adapter, said shroud having opposed inlets (46a) to an internal thread (49) for threadedly mating with the receptacle end of said hub as the receptacle end of said hub is fitted to the tapered inner receptacle end of said adapter core.

7. Apparatus of claim 1, wherein said first configuration of the first end of said adapter is not connectable to a needle assembly having a conventional luer.

8. Apparatus of claim 1, further comprising a filter (56) having a first end (60) with a configuration complementary to said given configuration of said hub and a second end (62) having the same said given configuration as said hub, said filter adapted to be connectably interposed between said needle assembly and said adapter by having its first end coupled to the receptacle end of said hub and its second end coupled to the first end of said adapter.

9. A method of coupling a needle assembly including a hub having a receptacle end and a needle extending from its closed end to an appropriate medicament fluid store or fluid line, comprising the steps of:
a) effecting a particular formation during the manufacturing process for providing a given configuration at the receptacle end of said hub;
b) providing an adapter having a first end with a first configuration complementary to said given configuration for mating with said hub at its receptacle end;
c) effecting a second configuration to a second end of said adapter, the first and second ends are connected by a through passage through a single molded piece of said adapter;
d) providing a fluid store or a fluid line having a receptacle end with a configuration complementary to said second configuration for mating with the second end of said adapter; and
e) providing a locking mechanism at said adapter to prevent the removal of the receptacle end of said fluid store or fluid line from the second end of said adapter once said fluid store or fluid line and said adapter are mated to each other as rotation in the direction that ordinarily would have uncoupled the receptacle end of said fluid store or fluid line from the second end of said adapter would cause the second end of said adapter to rotate in unison with the receptacle end of said fluid store or fluid line to thereby prevent said fluid store or fluid line and said adapter from disengaging.

10. Method of claim 9, wherein said step a further comprises the step of:
providing an integral formation at the receptacle end of said hub to form said given configuration so that said hub is adapted to mate only with a counterpart receptacle end that has a configuration complementary to said given configuration.

11. Method of claim 9, wherein said step b further comprises the steps of:
providing an adapter core and a shroud fittable about said adapter core as parts of said adapter, the adapter core being the single molded piece;
forming the first and second ends of said adapter at opposite ends of said adapter core;
forming at least one pawl on a circumferential outer surface of said adapter core and ramped stops circumferentially along an interior surface of said shroud;
rotatably fitting said shroud about said adapter core to form said adapter;
wherein once said fluid store or fluid line is mated to the second end of said adapter, the pawl of said adapter core would coact with the ramped stops of said shroud to enable said fluid store or fluid line to be rotatable only in a direction that does not allow said adapter and said fluid store or fluid line to be uncoupled from each other.

12. Method of claim 9, further comprising the step of:
connectably interposing a filter having a first end with a configuration complementary to said given configuration of said hub and a second end having the same said given configuration as said hub between said needle assembly and said adapter by coupling the first end of said filter to the receptacle end of said hub and the second end of said filter to the first end of said adapter to thereby effect a filtered fluid communication path between said needle and said adapter.

## Patentansprüche

1. Vorrichtung, umfassend:
eine Nadelanordnung (2) mit einem Ansatz (4) mit einer bestimmten Formation, die während des Herstellungsverfahrens herbeigeführt wurde, um eine vorgegebene Konfiguration an seinem Aufnahmeende (6) bereitzustellen, und einer sich von seinem geschlossenen Ende (8) erstreckenden Nadel (10);
einen Adapter (20) mit einem ersten Ende (36 und 46, 46a) mit einer ersten Konfiguration, die zur vorgegebenen Konfiguration komplementär ist, damit sie mit dem Ansatz an seinem Aufnahmeende zusammenpasst, wobei der Adapter ein zweites Ende (24) mit einer zweiten Konfiguration aufweist, wobei die ersten und zweiten Enden durch einen Durchgangskanal (40) durch ein einzelnes Formstück (32) des Adapters verbunden sind; und
einen Fluidspeicher (28) oder eine Fluidleitung mit einem Aufnahmeende (29) mit einer Konfiguration, die zur zweiten Konfiguration komplementär ist, um mit dem zweiten Ende des Adapters zusammenzupassen;
wobei der Adapter einen Arretiermechanismus (38, 52) umfasst, um die Entfernung des Aufnahmeendes (29) des Fluidspeichers (28) oder der Fluidleitung vom zweiten Ende (24) des Adapter zu verhindern, nachdem der Fluidspeicher oder die Fluidleitung und der Adapter zusammengefügt wurden, weil eine Drehung in die Richtung, die normalerweise das Aufnahmeende des Fluidspeichers vom zweiten Ende des Adapters gelöst hätte, dazu führen würde, dass sich das zweite Ende des Adapters gemeinsam mit dem Aufnahmeende des Fluidspeichers oder der Fluidleitung dreht, um so zu verhindern, dass sich der Fluidspeicher oder die Fluidleitung und der Adapter aus ihrem Eingriff lösen.

2. Vorrichtung nach Anspruch 1, wobei das Aufnahmeende des Ansatzes eine integrierte Formation (16a, 16b) zur Bildung der vorgegebenen Konfiguration aufweist, so dass der Ansatz dazu ausgelegt ist, nur mit einem Gegenstück zu dem Aufnahmeende zusammenzupassen, das eine Konfiguration aufweist, die zu der vorgegebenen Konfiguration komplementär ist.

3. Vorrichtung nach Anspruch 2, wobei die integrierte Formation des Aufnahmeendes des Ansatzes zwei Vorsprünge umfasst, die sich von dem Aufnahmeende des Ansatzes aus erstrecken.

4. Vorrichtung nach Anspruch 1, wobei der Adapter einen Kragen (34) umfasst, der um einen Adapterkern (32) herum angebracht ist, wobei der Adapterkern das einzelne Formstück ist, wobei die ersten und zweiten Enden (24, 36) des Adapters an entgegengesetzten Enden des Adapterkerns gebildet sind, wobei der Arretiermechanismus wenigstens eine Sperrklinke (38) aufweist, die auf einer äußeren Umfangsseite des Adapterkerns gebildet ist, um zusammen gegen rampenförmige Anschläge (42) zu wirken, die umfangsmäßig entlang einer Innenfläche des Kragens gebildet sind, so dass nach dem Zusammenfügen des Fluidspeichers oder der Fluidleitung mit dem zweiten Ende des Adapters der Fluidspeicher oder die Fluidleitung und der Adapter nicht mehr voneinander gelöst werden können.

5. Vorrichtung nach Anspruch 1, wobei die Nadelanordnung eine Epiduralnadel umfasst.

6. Vorrichtung nach Anspruch 1, wobei der Adapter einen Adapterkern mit einem Durchgangskanal (40) und einen um den Adapterkern herum angebrachten Kragen umfasst, wobei das erste Ende (36) des Adapterkerns ein verjüngtes inneres Aufnahmeende ist, welches das erste Ende des Adapters bildet, wobei der Kragen gegenüberliegende Einlässe (46a) zu einem Innengewinde (49) aufweist, für Gewindeeingriff mit dem Aufnahmeende des Ansatzes, wenn das Aufnahmeende des Ansatzes mit dem verjüngten inneren Aufnahmeende des Adapterkerns zusammengefügt wird.

7. Vorrichtung nach Anspruch 1, wobei die erste Konfiguration des ersten Endes des Adapters nicht mit einer Nadelanordnung verbunden werden kann, die einen herkömmlichen Luer-Adapter aufweist.

8. Vorrichtung nach Anspruch 1, ferner umfassend einen Filter (56) mit einem ersten Ende (60) mit einer Konfiguration, die zur vorgegebenen Konfiguration des Ansatzes komplementär ist, und mit einem zweiten Ende (62), das dieselbe vorgegebene Konfiguration wie der Ansatz aufweist, wobei der Filter dazu ausgelegt ist, verbindbar zwischen der Nadelanordnung und dem Adapter angeordnet zu werden, indem sein erstes Ende mit dem Aufnahmeende des Ansatzes verbunden wird und sein zweites Ende mit dem ersten Ende des Adapters verbunden wird.

9. Verfahren zur Verbindung einer Nadelanordnung, die einen Ansatz mit einem Aufnahmeende und einer sich von seinem geschlossenen Ende aus ersteckenden Nadel aufweist, mit einem angemessenen Medikamentenfluidspeicher oder einer Fluidleitung, das die folgenden Schritte umfasst:
a) Herbeiführen einer bestimmten Formation während des Herstellungsverfahrens, um eine vorgegebene Konfiguration am Aufnahmeende des Ansatzes bereitzustellen;
b) Bereitstellen eines Adapters mit einem ersten Ende mit einer ersten Konfiguration, die zur vorgegebenen Konfiguration komplementär ist, damit sie mit dem Ansatz an seinem Aufnahmeende zusammenpasst;
c) Herbeiführen einer zweiten Konfiguration an einem zweiten Ende des Adapters, wobei die ersten und zweiten Enden durch einen Durchgangskanal durch ein einzelnes Formstück des Adapters verbunden sind;
d) Bereitstellen eines Fluidspeichers oder einer Fluidleitung mit einem Aufnahmeende mit einer Konfiguration, die zur zweiten Konfiguration komplementär ist, um mit dem zweiten Ende des Adapters zusammenzupassen; und
e) Bereitstellen eines Arretiermechanismus an dem Adapter, um die Entfernung des Aufnahmeendes des Fluidspeichers oder der Fluidleitung vom zweiten Ende des Adapters zu verhindern, nachdem der Fluidspeicher oder die Fluidleitung und der Adapter zusammengefügt wurden, weil eine Drehung in die Richtung, die normalerweise das Aufnahmeende des Fluidspeichers oder der Fluidleitung vom zweiten Ende des Adapters gelöst hätte, dazu führen würde, dass sich das zweite Ende des Adapters gemeinsam mit dem Aufnahmeende des Fluidspeichers oder der Fluidleitung dreht, um so zu verhindern, dass sich der Fluidspeicher oder die Fluidleitung und der Adapter aus ihrem Eingriff lösen.

10. Verfahren nach Anspruch 9, wobei der Schritt a ferner den folgenden Schritt umfasst:
Bereitstellen einer integrierten Formation am Aufnahmeende des Ansatzes zur Bildung der vorgegebenen Konfiguration, so dass der Ansatz dazu ausgelegt ist, nur mit einem Gegenstück zu dem Aufnahmeende zusammenzupassen, das eine Konfiguration aufweist, die zu der vorgegebenen Konfiguration komplementär ist.

11. Verfahren nach Anspruch 9, wobei der Schritt b ferner die folgenden Schritte umfasst:
Bereitstellen eines Adapterkerns und eines Kragens, der um den Adapterkern herum angebracht werden kann, als Teile des Adapters, wobei der Adapterkern das einzelne Formstück ist;
Bilden der ersten und zweiten Enden des Adapters an entgegengesetzten Enden des Adapterkerns;
Bilden wenigstens einer Sperrklinke auf einer äußeren Umfangsseite des Adapterkerns und von rampenförmigen Anschlägen umfangsmäßig entlang einer Innenfläche des Kragens;
drehendes Anbringen des Kragens um den Adapterkern zur Bildung des Adapters;
wobei nach dem Zusammenfügen des Fluidspeichers oder der Fluidleitung mit dem zweiten Ende des Adapters die Sperrklinke des Adapterkerns mit den rampenförmigen Anschlägen des Kragens zusammenarbeiten würde, damit der Fluidspeicher oder die Fluidleitung nur in eine Richtung drehbar sind, die es nicht gestattet, dass der Adapter und der Fluidspeicher oder die Fluidleitung voneinander gelöst werden.

12. Verfahren nach Anspruch 9, ferner den folgenden Schritt umfassend:
verbindbares Anordnen eines Filters mit einem ersten Ende mit einer Konfiguration, die zur vorgegebenen Konfiguration des Ansatzes komplementär ist, und mit einem zweiten Ende, das dieselbe vorgegebene Konfiguration wie der Ansatz aufweist, zwischen der Nadelanordnung und dem Adapter, indem das erste Ende des Filters mit dem Aufnahmeende des Ansatzes verbunden wird und das zweite Ende des Filters mit dem ersten Ende des Adapters verbunden wird, um so einen gefilterten Fluidkommunikationsweg zwischen der Nadel und dem Adapter zu bewirken.

## Revendications

1. Appareil comprenant :
un ensemble formant aiguille (2) comprenant un pavillon (4) présentant une structure particulière produite lors du procédé de fabrication afin de créer une configuration donnée au niveau de son extrémité de réception (6) et une aiguille (10) s'étendant à partir de son extrémité fermée (8) ;
un adaptateur (20) comportant une première extrémité (36 et 46, 46a) présentant une première configuration complémentaire à ladite configuration donnée en vue d'un accouplement avec ledit pavillon au niveau de son extrémité de réception, ledit adaptateur comportant une seconde extrémité (24) présentant une seconde configuration, les première et seconde extrémités étant raccordées par un passage traversant (40) à travers une pièce moulée unique (32) dudit adaptateur ; et
un réservoir de fluide (28) ou un conduit de fluide comportant une extrémité de réception (29) présentant une configuration complémentaire à ladite seconde configuration en vue d'un accouplement avec la seconde extrémité dudit adaptateur ;
ledit adaptateur comprenant un mécanisme de verrouillage (38, 52) servant à empêcher le retrait de l'extrémité de réception (29) dudit réservoir de fluide (28) ou dudit conduit de fluide vis-à-vis de la seconde extrémité (24) dudit adaptateur une fois que ledit réservoir de fluide ou ledit conduit de fluide et ledit adaptateur ont été accouplés puisqu'une rotation dans le sens qui aurait désaccouplé d'ordinaire l'extrémité de réception dudit réservoir de fluide vis-à-vis de la seconde extrémité dudit adaptateur amènerait la seconde extrémité dudit adaptateur à tourner conjointement avec l'extrémité de réception dudit réservoir de fluide ou dudit conduit de fluide, de façon à empêcher ainsi un désaccouplement dudit réservoir de fluide ou dudit conduit de fluide et dudit adaptateur.

2. Appareil selon la revendication 1, dans lequel l'extrémité de réception dudit pavillon comporte une structure intégrée (16a, 16b) servant à former ladite configuration donnée de telle sorte que ledit pavillon puisse être accouplé uniquement avec une extrémité de réception correspondante qui présente une configuration complémentaire à ladite configuration donnée.

3. Appareil selon la revendication 2, dans lequel ladite structure intégrée de l'extrémité de réception dudit pavillon comprend deux protubérances s'étendant à partir de l'extrémité de réception dudit pavillon.

4. Appareil selon la revendication 1, dans lequel ledit adaptateur comprend une enveloppe (34) adaptée autour d'une partie centrale d'adaptateur (32), la partie centrale d'adaptateur étant la pièce moulée unique, les première et seconde extrémités (24, 36) dudit adaptateur étant formées au niveau d'extrémités opposées de ladite partie centrale d'adaptateur, dans lequel le mécanisme de verrouillage comprend au moins un cliquet (38) formé sur une surface circonférentielle extérieure de ladite partie centrale d'adaptateur et destiné à collaborer avec des butées inclinées (42) formées dans le sens de la circonférence le long d'une surface intérieure de ladite enveloppe de telle sorte qu'une fois que ledit réservoir de fluide ou ledit conduit de fluide a été accouplé avec la seconde extrémité dudit adaptateur, ledit réservoir de fluide ou ledit conduit de fluide et ledit adaptateur ne peuvent pas être désaccouplés.

5. Appareil selon la revendication 1, dans lequel ledit ensemble formant aiguille comprend une aiguille de péridurale.

6. Appareil selon la revendication 1, dans lequel ledit adaptateur comprend une partie centrale d'adaptateur comportant un canal traversant (40) et une enveloppe adaptée autour de ladite partie centrale d'adaptateur, la première extrémité (36) de ladite partie centrale d'adaptateur étant une extrémité de réception intérieure effilée qui forme la première extrémité dudit adaptateur, ladite enveloppe comportant des points d'entrée opposés (46a) menant à un filet intérieur (49) en vue d'un accouplement par vissage avec l'extrémité de réception dudit pavillon lorsque l'extrémité de réception dudit pavillon est adaptée sur l'extrémité de réception intérieure effilée de ladite partie centrale d'adaptateur.

7. Appareil selon la revendication 1, dans lequel ladite première configuration de la première extrémité dudit adaptateur ne peut pas être raccordée à un ensemble formant aiguille comportant un embout Luer conventionnel.

8. Appareil selon la revendication 1, comprenant en outre un filtre (56) comportant une première extrémité (60) présentant une configuration complémentaire à ladite configuration donnée dudit pavillon et une seconde extrémité (62) présentant la même dite configuration donnée que ledit pavillon, ledit filtre étant conçu pour être intercalé, de façon à permettre un raccordement, entre ledit ensemble formant aiguille et ledit adaptateur par l'accouplement de sa première extrémité avec l'extrémité de réception dudit pavillon et l'accouplement de sa seconde extrémité avec la première extrémité dudit adaptateur.

9. Procédé d'accouplement d'un ensemble formant aiguille, comprenant un pavillon comportant une extrémité de réception et une aiguille s'étendant à partir de son extrémité fermée, à un réservoir ou un conduit de fluide médicamenteux approprié, comprenant les étapes suivantes :
a) produire une structure particulière lors du procédé de fabrication afin de créer une configuration donnée au niveau de l'extrémité de réception dudit pavillon ;
b) prévoir un adaptateur comportant une première extrémité présentant une première configuration complémentaire à ladite configuration donnée en vue d'un accouplement avec ledit pavillon au niveau de son extrémité de réception ;
c) produire une seconde configuration à une seconde extrémité dudit adaptateur, les première et seconde extrémités étant raccordées par un passage traversant à travers une pièce moulée unique dudit adaptateur ;
d) prévoir un réservoir de fluide ou un conduit de fluide comportant une extrémité de réception présentant une configuration complémentaire à ladite seconde configuration en vue d'un accouplement avec la seconde extrémité dudit adaptateur ; et
e) prévoir un mécanisme de verrouillage au niveau dudit adaptateur afin d'empêcher le retrait de l'extrémité de réception dudit réservoir de fluide ou dudit conduit de fluide vis-à-vis de la seconde extrémité dudit adaptateur une fois que ledit réservoir de fluide ou ledit conduit de fluide et ledit adaptateur ont été accouplés puisqu'une rotation dans le sens qui aurait désaccouplé d'ordinaire l'extrémité de réception dudit réservoir de fluide ou dudit conduit de fluide vis-à-vis de la seconde extrémité dudit adaptateur amènerait la seconde extrémité dudit adaptateur à tourner conjointement avec l'extrémité de réception dudit réservoir de fluide ou dudit conduit de fluide, de façon à empêcher ainsi un désaccouplement dudit réservoir de fluide ou dudit conduit de fluide et dudit adaptateur.

10. Procédé selon la revendication 9, dans lequel l'étape a comprend en outre l'étape suivants :
prévoir une structure intégrée au niveau de l'extrémité de réception dudit pavillon afin de former ladite configuration donnée de telle sorte que ledit pavillon puisse être accouplé uniquement avec une extrémité de réception correspondante qui présente une configuration complémentaire à ladite configuration donnée.

11. Procédé selon la revendication 9, dans lequel l'étape b comprend en outre les étapes suivantes :
prévoir une partie centrale d'adaptateur et une enveloppe pouvant être adaptée autour de ladite partie centrale d'adaptateur en tant que parties dudit adaptateur, la partie centrale d'adaptateur étant la pièce moulée unique ;
former les première et seconde extrémités dudit adaptateur au niveau d'extrémités opposées de ladite partie centrale d'adaptateur ;
former au moins un cliquet sur une surface circonférentielle extérieure de ladite partie centrale d'adaptateur et des butées inclinées dans le sens de la circonférence le long d'une surface intérieure de ladite enveloppe ;
adapter à rotation ladite enveloppe autour de ladite partie centrale d'adaptateur de façon à former ledit adaptateur ;
dans lequel une fois que ledit réservoir de fluide ou ledit conduit de fluide a été accouplé avec la seconde extrémité dudit adaptateur, le cliquet de ladite partie centrale d'adaptateur collaborerait avec les butées inclinées de ladite enveloppe de telle sorte que ledit réservoir de fluide ou ledit conduit de fluide ne puisse effectuer une rotation que dans un sens qui ne permet pas le désaccouplement dudit adaptateur et dudit réservoir de fluide ou dudit conduit de fluide.

12. Procédé selon la revendication 9, comprenant en outre l'étape suivants :
intercaler, de façon à permettre un raccordement, un filtre, comportant une première extrémité présentant une configuration complémentaire à ladite configuration donnée dudit pavillon et une seconde extrémité présentant la même dite configuration donnée que ledit pavillon, entre ledit ensemble formant aiguille et ledit adaptateur par l'accouplement de la première extrémité dudit filtre avec l'extrémité de réception dudit pavillon et de la seconde extrémité dudit filtre avec la première extrémité dudit adaptateur, de façon à produire ainsi une voie de communication fluidique filtrée entre ladite aiguille et ledit adaptateur.
